(19) **Europäisches Patentamt / European Patent Office / Office européen des brevets**

(11) **EP 3 488 783 B1**

(12) **EUROPEAN PATENT SPECIFICATION**

(45) Date of publication and mention
of the grant of the patent:
**04.11.2020 Bulletin 2020/45**

(21) Application number: **17830889.6**

(22) Date of filing: **10.07.2017**

(51) Int Cl.:
**A61B 6/00** *(2006.01)*

(86) International application number:
**PCT/JP2017/025141**

(87) International publication number:
**WO 2018/016369 (25.01.2018 Gazette 2018/04)**

(54) **X-RAY PHASE DIFFERENCE IMAGING APPARATUS**

RÖNTGENPHASENDIFFERENZABBILDUNGSVORRICHTUNG

APPAREIL D'IMAGERIE À DIFFÉRENCE DE PHASE DE RAYONNEMENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.07.2016 JP 2016142604**

(43) Date of publication of application:
**29.05.2019 Bulletin 2019/22**

(73) Proprietors:
• **Shimadzu Corporation**
 **Kyoto 604-8511 (JP)**
• **Osaka University**
 **Suita-shi, Osaka 565-0871 (JP)**

(72) Inventors:
• **HORIBA, Akira**
 **Kyoto-shi**
 **Kyoto 604-8511 (JP)**
• **TANABE, Koichi**
 **Kyoto-shi**
 **Kyoto 604-8511 (JP)**
• **YOSHIMUTA, Toshinori**
 **Kyoto-shi**
 **Kyoto 604-8511 (JP)**
• **KIMURA, Kenji**
 **Kyoto-shi**
 **Kyoto 604-8511 (JP)**
• **KISHIHARA, Hiroyuki**
 **Kyoto-shi**
 **Kyoto 604-8511 (JP)**

• **WADA, Yukihisa**
 **Kyoto-shi**
 **Kyoto 604-8511 (JP)**
• **IZUMI, Takuro**
 **Kyoto-shi**
 **Kyoto 604-8511 (JP)**
• **SHIRAI, Taro**
 **Kyoto-shi**
 **Kyoto 604-8511 (JP)**
• **DOKI, Takahiro**
 **Kyoto-shi**
 **Kyoto 604-8511 (JP)**
• **SANO, Satoshi**
 **Kyoto-shi**
 **Kyoto 604-8511 (JP)**
• **SHIMURA, Takayoshi**
 **Suita-shi**
 **Osaka 565-0871 (JP)**
• **WATANABE, Heiji**
 **Suita-shi**
 **Osaka 565-0871 (JP)**
• **HOSOI, Takuji**
 **Suita-shi**
 **Osaka 565-0871 (JP)**

(74) Representative: **Müller-Boré & Partner**
 **Patentanwälte PartG mbB**
 **Friedenheimer Brücke 21**
 **80639 München (DE)**

(56) References cited:
 **WO-A1-2014/027289    JP-A- 2011 227 041**
 **JP-A- 2012 024 554    US-A1- 2011 243 300**
 **US-A1- 2012 163 537**

## Description

Technical Field

The present invention relates to an X-ray phase contrast imaging apparatus.

Background Art

Conventionally, an X-ray phase contrast imaging apparatus is known. Such an X-ray phase contrast imaging apparatus is disclosed in International Publication No. 2014/030115, for example.

International Publication No. 2014/030115 discloses an X-ray imaging system (X-ray phase contrast imaging apparatus) that images the inside of a subject using the phase contrast of X-rays that have passed through the subject. This X-ray imaging system can image a light element object and a soft tissue of a living body that are unlikely to absorb X-rays by imaging the inside of the subject using the phase contrast of the X-rays instead of the amount of absorption of the X-rays.

This X-ray imaging system includes an X-ray source, a source grating, a phase grating, an analyser grating, and a detector. The X-ray source, the source grating, the phase grating, the analyser grating, and the detector are disposed side by side in this order in the irradiation axis direction of the X-ray source.

In this X-ray imaging system, the phase grating is irradiated with X-rays that have passed through the source grating. When passing through the phase grating, the X-rays are diffracted, and a self-image of the phase grating is formed at a predetermined distance from the phase grating. This is called the Talbot effect. The predetermined distance at which the self-image is formed is called the Talbot distance. In this X-ray imaging system, after the self-image of the phase grating is superimposed on an absorption grating, the X-rays are detected by the detector. Based on the detection result, the inside of the subject is imaged. In this X-ray imaging system, the phase grating and the analyser grating are fixed such that the grating planes are parallel to each other.

US 2011/243300 A1 discloses an X-ray imaging system including first to third absorption gratings. Initially, the third absorption grating is disposed in a Z axis orthogonal to a detection surface of a FPD, and the position of the third absorption grating is adjusted in θx and θy directions based on a dose of X-rays having passed through the third absorption grating. Then, the first absorption grating is disposed in the Z axis so as to produce a moire pattern. The position of the first absorption grating is adjusted in the θx and θy directions so that a frequency of the moire pattern detected by the FPD becomes uniform. Then, the position of the first absorption grating is adjusted in a Z or θz direction so that the FPD loses the detection of the moiré pattern. After that, the second absorption grating is aligned in a like manner as the first absorption grating.

US 2012/163537 A1 discloses a radiographic apparatus including a cassette attachment/detachment detection unit that detects attachment and detachment of the radiation image detector, or a grid attachment/detachment detection unit that detects attachment and detachment of the first and second gratings.

Prior Art

Patent Document

Patent Document 1: International Publication No. 2014/030115

Summary of the Invention

Problem to be Solved by the Invention

In the X-ray imaging system described in International Publication No. 2014/030115, the phase grating and the analyser grating are fixed, and thus a relative position between the phase grating and the analyser grating cannot be adjusted. Therefore, when the phase grating and the analyser grating are fixed in a state where the relative position between the phase grating and the analyser grating is deviated from a design position, unintended Moire fringes occur due to a superposition of the self-image of the phase grating and the analyser grating. In this case, the unintended Moire fringes are detected by the detector, and thus there is a problem that the image quality of a captured image is deteriorated due to the unintended Moire fringes.

The present invention is defined by the independent claim 1. Preferred embodiments are defined in the dependent claims. The present invention has been proposed in order to solve the aforementioned problem, and an object of the present invention is to provide an X-ray phase contrast imaging apparatus capable of significantly reducing or preventing deterioration of the image quality of a captured image due to unintended Moire fringes.

Means for Solving the Problem

In order to attain the aforementioned object, an X-ray phase contrast imaging apparatus according to a first aspect of the present invention includes an X-ray source, a first grating irradiated with X-rays from the X-ray source to form a self-image, a second grating irradiated with the X-rays that have passed through the first grating, a detector that detects the X-rays that have passed through the second grating, an adjustment mechanism that adjusts a position of the first grating or a position of the second grating, and a controller that controls the adjustment mechanism to adjust a misalignment of the first grating or a misalignment of the second grating based on Moire fringes detected by the detector.

In the X-ray phase contrast imaging apparatus according to the first aspect of the present invention, as described above, the adjustment mechanism that adjusts the position of the first grating or the position of the second grating is provided. Furthermore, the controller that controls the adjustment mechanism to adjust the misalignment of the first grating or the misalignment of the second grating based on the Moire fringes detected by the detector is provided. Thus, even when unintended Moire fringes occur, the misalignment of the first grating or the misalignment of the second grating can be adjusted based on the occurring unintended Moire fringes. Consequently, unintended Moire fringes can be eliminated, and thus detection of unintended Moire fringes by the detector can be significantly reduced or prevented. Thus, deterioration of the image quality of a captured image due to unintended Moire fringes can be significantly reduced or prevented. In addition, the misalignment of the first grating or the misalignment of the second grating is automatically adjusted using the adjustment mechanism by the controller, and thus the burden on an operator required to adjust the misalignment of the first grating or the misalignment of the second grating can be reduced.

In the aforementioned X-ray phase contrast imaging apparatus according to the first aspect, the controller preferably controls the adjustment mechanism to adjust the misalignment of the first grating or the misalignment of the second grating such that the detector no longer detects the Moire fringes or a pitch of the Moire fringes becomes a predetermined pitch. According to this structure, when the misalignment of the first grating or the misalignment of the second grating is adjusted by the adjustment mechanism such that the detector no longer detects the Moire fringes, detection of unintended Moire fringes by the detector can be reliably significantly reduced or prevented. This adjustment is effective when a captured image is acquired by a fringe scanning method in which X-ray imaging is performed a plurality of times including a state without Moire fringes. When the misalignment of the first grating or the misalignment of the second grating is adjusted by the adjustment mechanism such that the pitch of the Moire fringes becomes the predetermined pitch, the pitch of the Moire fringes can be adjusted to a predetermined pitch suitable for X-ray imaging of a subject while detection of unintended Moire fringes by the detector is significantly reduced or prevented. This adjustment is effective when a captured image is acquired from a single Moire image (Moire fringes having a predetermined pitch suitable for X-ray imaging of the subject) by a Fourier transform method.

In the aforementioned X-ray phase contrast imaging apparatus according to the first aspect, the controller preferably controls the adjustment mechanism to adjust the misalignment of the first grating or the misalignment of the second grating in at least one of an irradiation axis direction of the X-rays, a rotation direction around the irradiation axis direction of the X-rays, and rotation directions around a first orthogonal direction and a second orthogonal direction orthogonal to each other in a plane orthogonal to the irradiation axis direction of the X-rays based on the Moire fringes. When the first grating or the second grating is misaligned in the irradiation axis direction of the X-rays, unintended Moire fringes having a pitch in a grating direction occur. Therefore, the misalignment of the first grating or the misalignment of the second grating in the irradiation axis direction of the X-rays is adjusted as described above such that the unintended Moire fringes having a pitch in the grating direction can be eliminated, and thus detection of the unintended Moire fringes having a pitch in the grating direction by the detector can be effectively significantly reduced or prevented. When the first grating or the second grating is misaligned in the rotation direction around the irradiation axis direction of the X-rays, unintended Moire fringes having a pitch in a direction different from the grating direction occur. Therefore, the misalignment of the first grating or the misalignment of the second grating in the rotation direction around the irradiation axis direction of the X-rays is adjusted as described above such that the unintended Moire fringes having a pitch in the direction different from the grating direction can be eliminated, and thus detection of the unintended Moire fringes having a pitch in the direction different from the grating direction by the detector can be effectively significantly reduced or prevented. When the first grating or the second grating is misaligned in the rotation direction around the first orthogonal direction or the rotation direction around the second orthogonal direction, unintended Moire fringes having a distortion occur. Therefore, the misalignment of the first grating or the misalignment of the second grating in the rotation directions around the first orthogonal direction and the second orthogonal direction is adjusted as described above such that the unintended Moire fringes having a distortion can be eliminated, and thus detection of the unintended Moire fringes having a distortion by the detector can be effectively significantly reduced or prevented.

In this case, the controller preferably acquires a pitch of the Moire fringes in a grating direction based on the Moire fringes, and acquires a misalignment amount of the first grating or a misalignment amount of the second grating in the irradiation axis direction of the X-rays based on the acquired pitch of the Moire fringes in the grating direction. When the first grating or the second grating is misaligned in the irradiation axis direction of the X-rays, as described above, the Moire fringes having a pitch in the grating direction occur. Therefore, as in this structure, the pitch of the Moire fringes in the grating direction is acquired such that the misalignment amount of the first grating or the misalignment amount of the second grating in the irradiation axis direction of the X-rays can be accurately acquired using the relationship between the pitch of the Moire fringes in the grating direction and the misalignment amount of the first grating or the misalignment amount of the second grating in the irradiation axis direction of the X-rays. Consequently, the misalignment of the first grating or the misalignment of the second grating in the irradiation axis direction of the X-rays can be accurately adjusted such that the unintended Moire fringes having a pitch in the grating direction are eliminated.

In the aforementioned structure in which the misalignment of the first grating or the misalignment of the second grating in the rotation direction around the irradiation axis direction of the X-rays is adjusted, the controller preferably acquires a pitch of the Moire fringes in a direction different from a grating direction based on the Moire fringes, and acquires a misalignment amount of the first grating or a misalignment amount of the second grating in the rotation direction around the irradiation axis direction of the X-rays based on the acquired pitch of the Moire fringes in the direction different from the grating direction. When the first grating or the second grating is misaligned in the rotation direction around the irradiation axis direction of the X-rays, as described above, the unintended Moire fringes having a pitch in the direction different from the grating direction occur. Therefore, as in this structure, the pitch of the Moire fringes in the direction different from the grating direction is acquired such that the misalignment amount of the first grating or the misalignment amount of the second grating in the rotation direction around the irradiation axis direction of the X-rays can be accurately acquired using the relationship between the pitch of the Moire fringes in the direction different from the grating direction and the misalignment amount of the first grating or the misalignment amount of the second grating in the rotation direction around the irradiation axis direction of the X-rays. Consequently, the misalignment of the first grating or the misalignment of the second grating in a direction different from the irradiation axis direction of the X-rays can be accurately adjusted such that the unintended Moire fringes having a pitch in the direction different from the grating direction are eliminated.

In the aforementioned structure in which the misalignment of the first grating or the misalignment of the second grating in the rotation directions around the first orthogonal direction and the second orthogonal direction is adjusted, the controller preferably controls the adjustment mechanism to adjust the misalignment of the first grating or the misalignment of the second grating in the rotation direction around the first orthogonal direction and the rotation direction around the second orthogonal direction so as to eliminate a distortion of the Moire fringes. When the first grating or the second grating is misaligned in the rotation direction around the first orthogonal direction or the rotation direction around the second orthogonal direction, as described above, unintended Moire fringes having a distortion occur. Therefore, as in this structure, the misalignment of the first grating or the misalignment of the second grating in the rotation direction around the first orthogonal direction and the rotation direction around the second orthogonal direction is adjusted so as to eliminate the distortion of the Moire fringes such that unintended Moire fringes that occur due to the misalignment of the first grating or the misalignment of the second grating in the rotation direction around the first orthogonal direction and the rotation direction around the second orthogonal direction can be easily eliminated.

An X-ray phase contrast imaging apparatus according to a second aspect of the present invention includes an X-ray source, a grating irradiated with X-rays from the X-ray source to form a self-image, a detector that detects the X-rays that have passed through the grating, an adjustment mechanism that adjusts a position of the grating or a position of the detector, and a controller that controls the adjustment mechanism to adjust a misalignment of the grating or a misalignment of the detector based on Moire fringes detected by the detector.

In the X-ray phase contrast imaging apparatus according to the second aspect of the present invention, as described above, the adjustment mechanism that adjusts the position of the grating or the position of the detector is provided. Furthermore, the controller that controls the adjustment mechanism to adjust the misalignment of the grating or the misalignment of the detector based on the Moire fringes detected by the detector is provided. Thus, even when unintended Moire fringes occur, the misalignment of the grating or the misalignment of the detector can be adjusted based on the occurring unintended Moire fringes. Consequently, the unintended Moire fringes can be eliminated, and thus detection of unintended Moire fringes by the detector can be significantly reduced or prevented. Thus, similarly to the case of the X-ray phase contrast imaging apparatus according to the first aspect, deterioration of the image quality of a captured image due to unintended Moire fringes can be significantly reduced or prevented.

An X-ray phase contrast imaging apparatus according to a third aspect of the present invention includes an X-ray source, a first grating irradiated with X-rays from the X-ray source to form a self-image, a detector that detects the X-rays that have passed through at least the first grating, an adjustment mechanism that adjusts a relative position between the first grating and a second grating irradiated with the X-rays that have passed through the first grating or a relative position between the first grating and the detector, and a controller that controls the adjustment mechanism to adjust the relative position between the first grating and the second grating or the relative position between the first grating and the detector based on Moire fringes detected by the detector.

In the X-ray phase contrast imaging apparatus according to the third aspect of the present invention, as described above, the adjustment mechanism that adjusts the relative position between the first grating and the second grating irradiated with the X-rays that have passed through the first grating or the relative position between the first grating and the detector is provided. Furthermore, the controller that controls the adjustment mechanism to adjust the relative position between the first grating and the second grating or the relative position between the first grating and the detector based on the Moire fringes detected by the detector is provided. Thus, even when unintended Moire fringes occur, the relative position between the first grating and the second grating or the relative position between the first grating and the detector can be adjusted based on the Moire fringes detected by the detector. Consequently, the unintended Moire fringes can be eliminated, and thus detection of unintended Moire fringes by the detector can be significantly reduced or prevented. Thus, similarly to the case of the X-ray phase contrast imaging apparatus according to the first aspect, deterioration of the image quality of a captured image due to unintended Moire fringes can be significantly reduced or prevented.

Effect of the Invention

As described above, according to the present invention, the X-ray phase contrast imaging apparatus capable of significantly reducing or preventing deterioration of the image quality of a captured image due to unintended Moire fringes can be provided.

Brief Description of the Drawings

Fig. 1 is a diagram showing the overall structure of an X-ray phase contrast imaging apparatus according to a first embodiment of the present invention.
Fig. 2 is a diagram showing an X-ray source, a third grating, a first grating, a second grating, and a detector of the X-ray phase contrast imaging apparatus according to the first embodiment.
Fig. 3 is a diagram illustrating adjustment of the position of the first grating by an adjustment mechanism of the X-ray phase contrast imaging apparatus according to the first embodiment.
Fig. 4 is a diagram illustrating occurrence of unintended Moire fringes when the first grating or the second grating is misaligned in a direction Z.
Fig. 5 is a diagram illustrating occurrence of unintended Moire fringes when the first grating and the second grating are misaligned in a rotation direction around the direction Z.
Fig. 6 is a diagram illustrating occurrence of unintended Moire fringes when the first grating is misaligned in a rotation direction around a direction Y.
Fig. 7 is a diagram illustrating adjustment of the misalignment of the first grating in the rotation direction around the direction Y in the case shown in Fig. 6.
Fig. 8 is a flowchart illustrating grating position adjustment processing by the X-ray phase contrast imaging apparatus according to the first embodiment.
Fig. 9 is a diagram showing the overall structure of an X-ray phase contrast imaging apparatus according to a first modified example of the first embodiment of the present invention.
Fig. 10 is a diagram showing the overall structure of an X-ray phase contrast imaging apparatus according to a second embodiment of the present invention.
Fig. 11 is a diagram showing the overall structure of an X-ray phase contrast imaging apparatus according to a second modified example of the first embodiment of the present invention.
Fig. 12 is a diagram showing the overall structure of an X-ray phase contrast imaging apparatus according to a third modified example of the first embodiment of the present invention.
Fig. 13 is a diagram showing the overall structure of an X-ray phase contrast imaging apparatus according to a first modified example of the second embodiment of the present invention.

Modes for Carrying Out the Invention

Embodiments embodying the present invention are hereinafter described on the basis of the drawings.

[First Embodiment]

(Structure of X-ray Phase Contrast Imaging Apparatus)

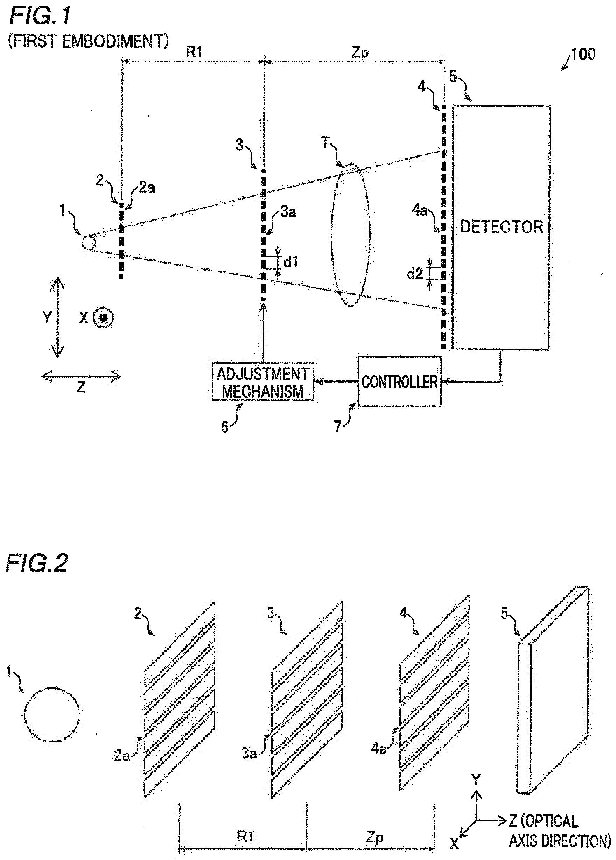

The structure of an X-ray phase contrast imaging apparatus 100 according to a first embodiment of the present invention is described with reference to Figs. 1 to 3.

As shown in Fig. 1, the X-ray phase contrast imaging apparatus 100 is an apparatus that images the inside of a subject T using the phase contrast of X-rays that have passed through the subject T. Furthermore, the X-ray phase contrast imaging apparatus 100 is an apparatus that images the inside of the subject T using the Talbot effect. For example, in medical applications, the X-ray phase contrast imaging apparatus 100 can be used to image the inside of the subject T as a living body. Furthermore, for example, in non-destructive inspection applications, the X-ray phase contrast imaging apparatus 100 can be used to image the inside of the subject T as an object.

As shown in Figs. 1 and 2, the X-ray phase contrast imaging apparatus 100 includes an X-ray source 1, a third grating 2, a first grating 3, a second grating 4, a detector 5, an adjustment mechanism 6, and a controller 7. The X-ray phase contrast imaging apparatus 100 performs X-ray imaging by a Talbot-Lau interferometer using the X-ray source 1, the third grating 2, the first grating 3, the second grating 4, and the detector. The X-ray source 1, the third grating 2, the first grating 3, the second grating 4, and the detector 5 are disposed side by side in this order in an X-ray irradiation axis direction (optical axis direction, direction Z).

In this specification, the X-ray irradiation axis direction is defined as the direction Z, and directions orthogonal to each other in a plane orthogonal to the direction Z are defined as a direction X and a direction Y, respectively. The direction X is an example of a "first orthogonal direction" in the claims. The direction Y is an example of a "second orthogonal direction" in the claims.

The X-ray source 1 generates X-rays when a high voltage is applied thereto and radiates the generated X-rays.

The third grating 2 is a diffraction grating (absorption grating, so-called multi slit) that changes the intensity of the passing X-rays. The third grating 2 includes a plurality of slits 2a arrayed at a predetermined pitch in the direction Y orthogonal to the direction Z. The slits 2a each extend in the direction X orthogonal to the direction Z.

The third grating 2 is disposed between the X-ray source 1 and the first grating 3, and X-rays are radiated thereto from the X-ray source 1. The third grating 2 is provided to increase the coherence of the X-rays radiated from the X-ray source 1 with the Lau effect. The third grating 2 allows the X-rays that have passed through the respective slits 2a to function as line light sources corresponding to the positions of the respective slits 2a. Thus, the third grating 2 can increase the coherence of the X-rays that have passed through the third grating 2.

The first grating 3 is a diffraction grating (phase grating) that changes the phase of the passing X-rays. The first grating 3 includes a plurality of slits 3a arrayed at a pitch d1 in the direction Y orthogonal to the direction Z. The slits 3a each extend in the direction X orthogonal to the direction Z.

The first grating 3 is disposed between the third grating 2 and the second grating 4, and the X-rays that have passed through the third grating 2 are radiated thereto. The first grating 3 is designed to be disposed at a position away from the third grating 2 by a distance R1. The first grating 3 is provided to form a self-image with the Talbot effect. When X-rays having coherence pass through a grating in which slits are provided, a grating image (self-image) is formed at a position away from the grating by a predetermined distance (Talbot distance Zp described below). This is called the Talbot effect. The self-image is an interference fringe caused by X-ray interference. In the X-ray phase contrast imaging apparatus 100, the third grating 2 is provided to increase the coherence of the X-rays such that the self-image of the first grating 3 due to the Talbot effect can be more reliably formed.

The second grating 4 is a diffraction grating (absorption grating) that changes the intensity of the passing X-rays. The second grating 4 includes a plurality of slits 4a arrayed at a pitch d2 in the direction Y orthogonal to the direction Z. The slits 4a each extend in the direction X orthogonal to the direction Z.

The second grating 4 is disposed between the first grating 3 and the detector 5, and the X-rays that have passed through the first grating 3 are radiated thereto. The second grating 4 is designed to be disposed at a position away from the first grating 3 by the Talbot distance Zp. The second grating 4 interferes with the self-image of the first grating 3 to form Moire fringes.

The Talbot distance Zp is expressed by the following formula (1) when d1 represents the pitch of the first grating 3, λ represents the wavelength of the X-rays radiated from the X-ray source 1, R1 represents the distance between the third grating 2 and the first grating 3, and p represents Talbot order.

[Mathematical Formula 1]

$$Zp = p \frac{d1^2}{\lambda} \frac{R1}{R1 - p\frac{d1^2}{\lambda}} \quad \cdots (1)$$

The pitch d3 of the self-image of the first grating 3 is expressed by the following formula (2).

[Mathematical Formula 2]

$$d3 = \frac{R1 + Zp}{R1} d1 \quad \cdots (2)$$

In the X-ray phase contrast imaging apparatus 100, the pitch d2 of the slits 4a of the second grating 4 is designed to be substantially the same as the pitch d3 of the self-image of the first grating 3. According to the first embodiment, the subject T is placed between the first grating 3 and the second grating 4 at the time of imaging the subject T. In this case, the second grating 4 is irradiated with the X-rays that have passed through the subject T.

The detector 5 detects the X-rays and converts the detected X-rays into an electric signal (detection signal). The detector 5 outputs the detection signal to the controller 7. The detector 5 is an FPD (Flat Panel Detector), for example. The detector 5 includes a plurality of detection elements (not shown). The plurality of detection elements are disposed side by side in the direction X and the direction Y at a predetermined pitch.

The pitch of the detection elements is larger than the pitch d3 of the self-image of the first grating 3. In this case, it is difficult for the detector 5 to directly detect the self-image of the first grating 3, and thus in the X-ray phase contrast imaging apparatus 100, the second grating 4 that interferes with the self-image of the first grating 3 to form Moire fringes is provided, and Moire fringes due to a superposition of the self-image of the first grating 3 and the second grating 4 are detected by the detector 5. The pitch of the Moire fringes due to the superposition of the self-image of the first grating 3 and the second grating 4 is sufficiently larger than the pitch of the detection elements, and thus the Moire fringes due to the superposition of the self-image of the first grating 3 and the second grating 4 can be detected by the detector 5.

The adjustment mechanism 6 adjusts the position of the first grating 3 based on a control signal from the controller 7. In other words, the adjustment mechanism 6 adjusts a relative position between the first grating 3 and the second grating 4 based on the control signal from the controller 7.

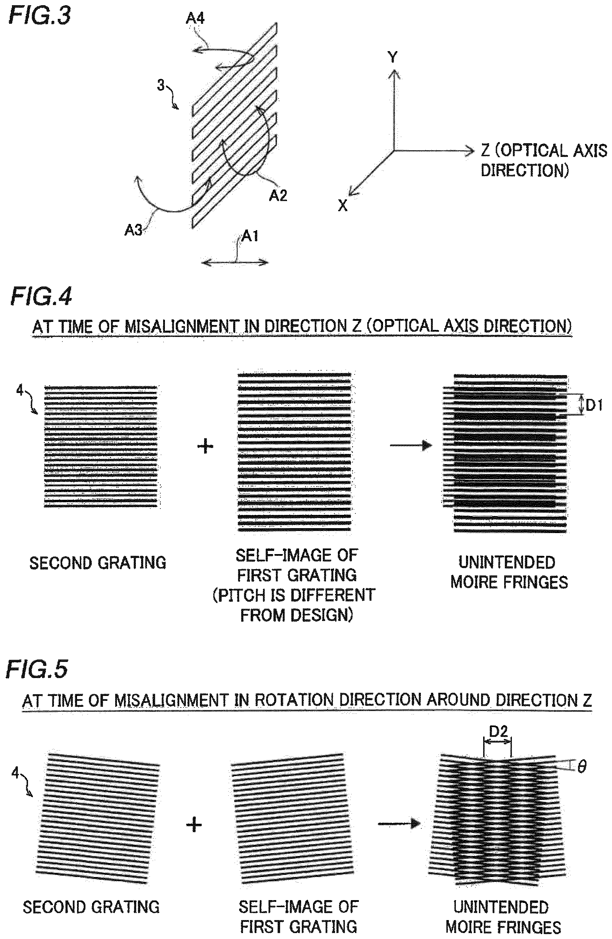

As shown in Fig. 3, the adjustment mechanism 6 can move the first grating 3 in an arrow A1 direction along the direction Z. Thus, the adjustment mechanism 6 can adjust the position of the first grating 3 in the direction Z. The adjustment mechanism 6 can adjust the position of the first grating 3 in the direction Z at a distance of 10 μm or more and 10 mm or less, for example.

The adjustment mechanism 6 can rotate the first grating 3 in an arrow A2 direction, which is a rotation direction around the direction Z. Thus, the adjustment mechanism 6 can adjust the position (tilt) of the first grating 3 in the rotation direction around the direction Z. The adjustment mechanism 6 can adjust the position of the first grating 3 in the rotation direction around the direction Z at an angular interval of 0.001 degrees or more and 1 degree or less, for example.

The adjustment mechanism 6 can rotate the first grating 3 in an arrow A3 direction, which is a rotation direction around the direction X. Thus, the adjustment mechanism 6 can adjust the position of the first grating 3 in the rotation direction around the direction X. The adjustment mechanism 6 can rotate the first grating 3 in an arrow A4 direction, which is a rotation direction around the direction Y. Thus, the adjustment mechanism 6 can adjust the position of the first grating 3 in the rotation direction around the direction Y. The adjustment mechanism 6 can adjust the position of the first grating 3 in the rotation direction around the direction X and the position of the first grating 3 in the rotation direction around the direction Y at an angular interval of 0.01 degrees or more and 5 degrees or less, for example.

The adjustment mechanism 6 is an electric positioning stage using a stepping motor or a piezo actuator, for example.

The controller 7 includes a CPU (Central Processing Unit), and controls the adjustment mechanism 6 based on the detection result of the detector 5. Control of the adjustment mechanism 6 by the controller 7 is described below in detail.

(Occurrence of Unintended Moire Fringes)

Occurrence of unintended Moire fringes in the X-ray phase contrast imaging apparatus 100 is described with reference to Figs. 4 to 6. In the X-ray phase contrast imaging apparatus 100, unintended Moire fringes occur due to a misalignment between the gratings.

Fig. 4 shows unintended Moire fringes that occur when the first grating 3 is misaligned in the direction Z from the distance R1, which is a design value. In this case, as can be seen from the aforementioned formula (2), the distance R1 is deviated from the design value, and thus the pitch d3 of the self-image of the first grating 3 calculated using the distance R1 deviated from the design value becomes a value different from the design value. Therefore, as shown in Fig. 4, the pitch d3 of the self-image of the first grating 3 does not match the pitch d2 of the second grating 4, and when the self-image of the first grating 3 is superimposed on the second grating 4, unintended Moire fringes having a pitch D1 in a grating direction (direction in which the slits are arrayed, direction Y) occur.

Fig. 5 shows unintended Moire fringes that occur when the first grating 3 is misaligned (tilted) in the rotation direction around the direction Z from the design value. In Fig. 5, for ease of understanding, the second grating 4 is tilted. In this case, the parallelism of the self-image of the first grating 3 and the parallelism of the second grating 4 are different from each other (directions in which the slits extend intersect with each other), and thus when the self-image of the first grating 3 is superimposed on the second grating 4, unintended Moire fringes having a pitch D2 in a direction different from the grating direction occur.

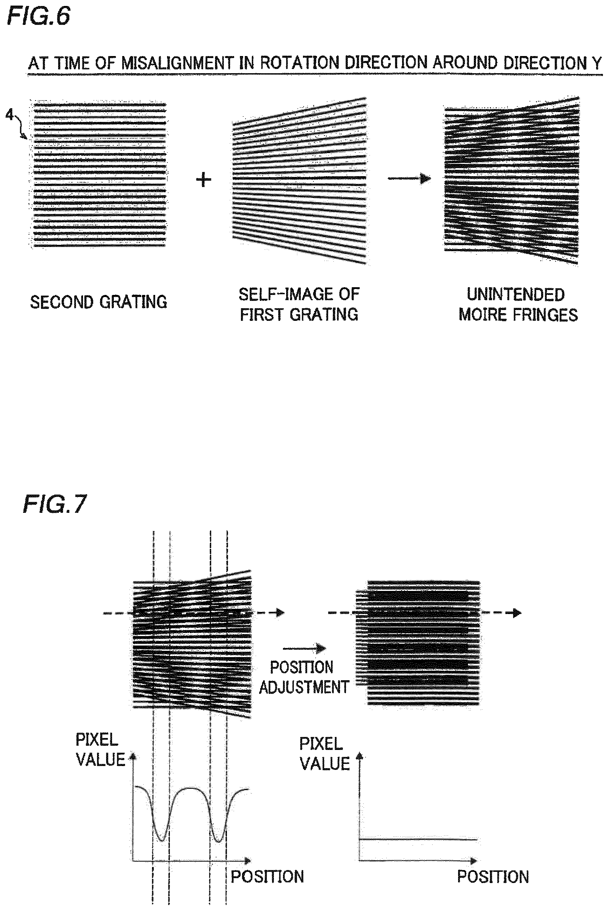

Fig. 6 shows unintended Moire fringes that occur when the first grating 3 is misaligned (tilted) in the rotation direction around the direction Y from the design value. In this case, the pitch d3 of the self-image of the first grating 3 varies within the plane of the self-image, and thus when the self-image of the first grating 3 is superimposed on the second grating 4, unintended Moire fringes having a distortion occur. Even when the first grating 3 is misaligned in the rotation direction around the direction X, unintended Moire fringes having a distortion occur similarly to the case shown in Fig. 6.

(Structure of Position Adjustment of Grating)

According to the first embodiment, the controller 7 controls the adjustment mechanism 6 to adjust the misalignment of the first grating 3 based on the unintended Moire fringes detected by the detector 5. In other words, the controller 7 controls the adjustment mechanism 6 to adjust the relative position between the first grating 3 and the second grating 4 based on the unintended Moire fringes detected by the detector 5. Note that unintended Moire fringes are detected in a state where the subject T is not placed.

According to the first embodiment, the X-ray phase contrast imaging apparatus 100 acquires a captured image by a fringe scanning method in which X-ray imaging is performed a plurality of times including a state without Moire fringes. Therefore, the controller 7 controls the adjustment mechanism 6 to adjust the misalignment of the first grating 3 such that the detector 5 no longer detects unintended Moire fringes.

According to the first embodiment, the controller 7 controls the adjustment mechanism 6 to adjust the misalignment of the first grating 3 in the direction Z, the rotation direction around the direction Z, the rotation direction around the direction X, and the rotation direction around the direction Y based on the unintended Moire fringes detected by the detector 5.

First, adjustment of the misalignment of the first grating 3 in the direction Z is described.

When the first grating 3 is misaligned in the direction Z, unintended Moire fringes having a pitch D1 in the grating direction occur, as shown in Fig. 4. Therefore, according to the first embodiment, the controller 7 acquires the pitch D1 of the Moire fringes in the grating direction based on the unintended Moire fringes detected by the detector 5. Then, the controller 7 acquires the misalignment amount of the first grating 3 in the direction Z based on the acquired pitch D1 of the Moire fringes in the grating direction. Acquisition of the misalignment amount of the first grating 3 in the direction Z based on the pitch D1 is hereinafter described.

The pitch D1 of the Moire fringes in the grating direction is expressed by the following formula (3) when $\Delta d$ represents the absolute value of a difference (error) between the pitch d2 of the second grating 4 and the pitch d3 of the self-image of the first grating 3.
[Mathematical Formula 3]

$$D1 = \frac{d2^2}{\Delta d} \quad \cdots (3)$$

$\Delta d$ is expressed by the following formula (4).
[Mathematical Formula 4]

$$\Delta d = |d2 - d3| \quad \cdots (4)$$

First, the controller 7 acquires (reads) the pitch D1 of the Moire fringes in the grating direction by image processing. Then, the controller 7 acquires the pitch d3 of the self-image of the first grating 3 using the acquired pitch D1 of the Moire fringes in the grating direction and the aforementioned formulas (3) and (4). The acquired pitch d3 of the self-image of the first grating 3 is a value different from the design value.

Then, the controller 7 acquires a distance R1p between the third grating 2 and the first grating 3 in a misaligned state using the following formula (5) obtained by modifying the aforementioned formula (2).
[Mathematical Formula 5]

$$R1p = \frac{Zp \cdot d1}{d3 - d1} \quad \cdots (5)$$

In the formula (5), whereas, the pitch d1 of the first grating 3 and the pitch d3 of the self-image of the first grating 3 are known values, the Talbot distance Zp changes according to the value of R1, and thus it is unknown. Therefore, according to the first embodiment, a design value calculated from the aforementioned formula (1) is used as the value of the Talbot distance Zp when the distance R1p is acquired using the formula (5). At this time, the design value is also used as R1 in the formula (1).

Then, the controller 7 acquires the misalignment amount $\Delta R$ of the first grating 3 in the direction Z by acquiring a difference between R1, which is the design value of the distance between the third grating 2 and the first grating 3, and the distance R1p between the third grating 2 and the first grating 3 in a misaligned state using the following formula (6).
[Mathematical Formula 6]

$$R1 - R1p = \Delta R \quad \cdots (6)$$

Then, the controller 7 controls the adjustment mechanism 6 to move the first grating 3 in the direction A1 (see Fig. 3) by the misalignment amount $\Delta R$ so as to adjust the misalignment of the first grating 3 in the direction Z. When the misalignment amount $\Delta R$ is a negative value, the first grating 3 is moved in the direction A1 toward the detector 5 by the adjustment mechanism 6. When the misalignment amount $\Delta R$ is a positive value, the first grating 3 is moved in the direction A1 opposite to the direction toward the detector 5 by the adjustment mechanism 6.

After adjusting the misalignment of the first grating 3 in the direction Z, the controller 7 performs X-ray imaging again and confirms whether or not unintended Moire fringes occur. According to the first embodiment, whether or not unintended Moire fringes occur is determined by whether or not Moire fringes are detected by the detector 5. When unintended Moire fringes occur, the misalignment of the first grating 3 in the direction Z is adjusted again.

Next, adjustment of the misalignment of the first grating 3 in the rotation direction around the direction Z is described.

When the first grating 3 is misaligned in the rotation direction around the direction Z, unintended Moire fringes having a pitch D2 in the direction different from the grating direction occur, as shown in Fig. 5. Therefore, according to the first embodiment, the controller 7 acquires the pitch D2 of the Moire fringes in the direction different from the grating direction based on the unintended Moire fringes detected by the detector 5. Then, the controller 7 acquires the misalignment amount of the first grating 3 in the rotation direction around the direction Z based on the acquired pitch D2 of the Moire fringes in the direction different from the grating direction. Acquisition of the misalignment amount of the first grating 3 in the rotation direction around the direction Z based on the pitch D2 is hereinafter described.

The pitch D2 of the Moire fringes in the direction different from the grating direction is expressed by the following formula (7) when an angle formed by the self-image of the first grating 3 and the second grating 4 is $\theta$.
[Mathematical Formula 7]

$$D2 = \frac{d2}{2\sin(\frac{\theta}{2})} \quad \cdots (7)$$

The angle θ formed by the self-image of the first grating 3 and the second grating 4 is the misalignment amount of the first grating 3 in the rotation direction around the direction Z. Therefore, the controller 7 acquires the misalignment amount θ of the first grating 3 in the rotation direction around the direction Z using the aforementioned formula (7).

Then, the controller 7 controls the adjustment mechanism 6 to rotate the first grating 3 in the direction A2 (see Fig. 3) by the misalignment amount θ so as to adjust the misalignment of the first grating 3 in the rotation direction around the direction Z.

After adjusting the misalignment of the first grating 3 in the rotation direction around the direction Z, the controller 7 performs X-ray imaging again and confirms whether or not unintended Moire fringes occur. When unintended Moire fringes occur, the misalignment of the first grating 3 in the rotation direction around the direction Z is adjusted again.

Next, adjustment of the misalignment of the first grating 3 in the rotation direction around the direction X and the rotation direction around the direction Y is described. Here, adjustment of the misalignment of the first grating 3 in the rotation direction around the direction Y is described as an example with reference to Figs. 6 and 7. The misalignment of the first grating 3 in the rotation direction around the direction X can be adjusted similarly.

When the misalignment of the first grating 3 occurs in the rotation direction around the direction Y, unintended Moire fringes having a distortion occur, as shown in Fig. 6. Therefore, according to the first embodiment, the controller 7 controls the adjustment mechanism 6 to adjust the misalignment of the first grating 3 in the rotation direction around the direction Y so as to eliminate the distortion of the unintended Moire fringes based on pixel values of the unintended Moire fringes.

As shown in Fig. 7, when Moire fringes having a distortion occur, a pixel value at each position of the Moire fringes along a direction orthogonal to the grating direction varies. On the other hand, when Moire fringes having a distortion do not occur, a pixel value at each position of the Moire fringes along the direction orthogonal to the grating direction is substantially constant.

Therefore, the controller 7 acquires the pixel value at each position of the Moire fringes along the direction orthogonal to the grating direction. Then, the controller 7 controls the adjustment mechanism 6 to rotate the first grating 3 in the arrow A4 direction (see Fig. 3) such that the pixel value at each position of the Moire fringes along the direction orthogonal to the grating direction becomes substantially constant so as to adjust the misalignment of the first grating 3 in the rotation direction around the direction Y. At this time, the controller 7 controls the adjustment mechanism 6 to adjust the misalignment of the first grating 3 in the rotation direction around the direction Y so as to eliminate the distortion of the unintended Moire fringes while performing X-ray imaging.

(Grating Position Adjustment Processing)

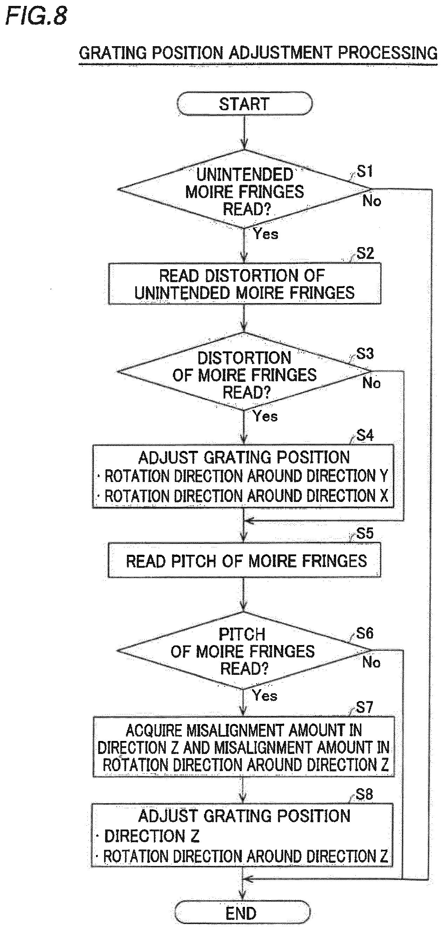

Next, grating position adjustment processing in the X-ray phase contrast imaging apparatus 100 is described based on a flowchart with reference to Fig. 8. The processing of the flowchart is performed by the controller 7.

When X-ray imaging is performed in a state where the subject T is not placed, first in step S1, it is determined whether or not unintended Moire fringes have been read (there are unintended Moire fringes) by image processing. According to the first embodiment, when Moire fringes are not detected by the detector 5, it is determined that unintended Moire fringes have not been read. In this case, it is not necessary to adjust the position of the first grating 3, and thus the grating position adjustment processing is terminated.

When Moire fringes are detected by the detector 5 in step S1, it is determined that unintended Moire fringes have been read, and the processing advances to step S2.

Then, in step S2, the distortion of the Moire fringes is read by image processing.

Then, in step S3, it is determined whether or not the distortion of the Moire fringes has been read (there is a distortion in the Moire fringes). That is, it is determined whether or not unintended Moire fringes having a distortion as shown in Fig. 6 have been read. When it is determined that the distortion of the Moire fringes has been read, the processing advances to step S4.

Then, in step S4, the misalignment of the first grating 3 in the rotation direction around the direction Y and the rotation direction around the direction X is adjusted using the adjustment mechanism 6 so as to eliminate the read distortion of the Moire fringes. Thereafter, the processing advances to step S5.

When it is determined that the distortion of the Moire fringes has not been read in step S3, it is not necessary to adjust the misalignment of the first grating 3 in the rotation direction around the direction Y and the rotation direction around the direction X, the processing advances to step S5 without performing step S4.

Then, in step S5, the pitch of the Moire fringes is read. In the grating position adjustment processing, the distortion of the Moire fringes is read before the pitch of the Moire fringes. This is because it is difficult to read the pitch of the Moire fringes unless Moire fringes having a distortion are adjusted when the Moire fringes having a distortion occur.

Then, in step S5, both the pitch D1 (see Fig. 4) of the Moire fringes in the grating direction and the pitch D2 (see Fig. 5) of the Moire fringes in the direction different from the grating direction are read.

Then, in step S6, it is determined whether or not the pitch of the Moire fringes has been read (acquired). When it is determined that at least one of the pitch D1 of the Moire fringes in the grating direction and the pitch D2 of the Moire fringes in the direction different from the grating direction has been read (acquired), the processing advances to step S7.

Then, when the pitch D1 of the Moire fringes in the grating direction is acquired in step S6, the misalignment amount $\Delta R$ of the first grating 3 in the direction Z is acquired using the aforementioned formulas (3) to (6) in step S7. When the pitch D2 of the Moire fringes in the direction different from the grating direction is acquired in step S6, the misalignment amount $\theta$ of the first grating 3 in the rotation direction around the direction Z is acquired using the aforementioned formula (7) in step S7.

Then, in step S8, the misalignment of the first grating 3 in the direction Z is adjusted based on the misalignment amount $\Delta R$ of the first grating 3 in the direction Z acquired in step S7. Furthermore, in step S8, the misalignment of the first grating 3 in the rotation direction around the direction Z is adjusted based on the misalignment amount $\theta$ of the first grating 3 in the rotation direction around the direction Z acquired in step S7. When unintended Moire fringes are not eliminated by one adjustment, the processing in step S5 to step S8 may be repeated until unintended Moire fringes are eliminated. Then, the grating position adjustment processing is terminated.

(Effects of First Embodiment)

According to the first embodiment, the following effects are achieved.

According to the first embodiment, as described above, the adjustment mechanism 6 that adjusts the position of the first grating 3 is provided. Furthermore, the controller 7 that controls the adjustment mechanism 6 to adjust the misalignment of the first grating 3 based on the Moire fringes detected by the detector 5 is provided. Thus, even when unintended Moire fringes occur, the misalignment of the first grating 3 can be adjusted based on the occurring unintended Moire fringes. Consequently, unintended Moire fringes can be eliminated, and thus detection of unintended Moire fringes by the detector 5 can be significantly reduced or prevented. Thus, deterioration of the image quality of the captured image due to unintended Moire fringes can be significantly reduced or prevented. In addition, the misalignment of the first grating 3 is automatically adjusted using the adjustment mechanism 6 by the controller 7, and thus the burden on an operator required to adjust the misalignment of the first grating 3 can be reduced.

According to the first embodiment, as described above, the controller 7 controls the adjustment mechanism 6 to adjust the misalignment of the first grating 3 such that the detector 5 no longer detects the Moire fringes. Thus, when the misalignment of the first grating 3 is adjusted by the adjustment mechanism 6 such that the detector 5 no longer detects the Moire fringes, detection of unintended Moire fringes by the detector 5 can be reliably significantly reduced or prevented. This adjustment is effective when a captured image is acquired by the fringe scanning method in which X-ray imaging is performed a plurality of times including a state without Moire fringes as in the X-ray phase contrast imaging apparatus 100 according to the first embodiment.

According to the first embodiment, as described above, the controller 7 controls the adjustment mechanism 6 to adjust the misalignment of the first grating 3 in the X-ray irradiation axis direction (direction Z), the rotation direction around the X-ray irradiation axis direction, and the rotation directions around the direction X and the direction Y orthogonal to each other in the plane orthogonal to the X-ray irradiation axis direction based on the Moire fringes. When the first grating 3 is misaligned in the X-ray irradiation axis direction, the unintended Moire fringes having a pitch D1 in the grating direction occur, as shown in Fig. 4. Therefore, the misalignment of the first grating 3 in the X-ray irradiation axis direction is adjusted as described above such that the unintended Moire fringes having a pitch D1 in the grating direction can be eliminated, and thus detection of the unintended Moire fringes having a pitch D1 in the grating direction by the detector 5 can be effectively significantly reduced or prevented. When the first grating 3 is misaligned in the rotation direction around the X-ray irradiation axis direction, the unintended Moire fringes having a pitch D2 in the direction different from the grating direction occur, as shown in Fig. 5. Therefore, the misalignment of the first grating 3 in the rotation direction around the X-ray irradiation axis direction is adjusted as described above such that the unintended Moire fringes having a pitch D2 in the direction different from the grating direction can be eliminated, and thus detection of the unintended Moire fringes having a pitch D2 in the direction different from the grating direction by the detector 5 can be effectively significantly reduced or prevented. When the first grating 3 is misaligned in the rotation direction around the direction X or the rotation direction around the direction Y, the unintended Moire fringes having a distortion occur, as shown in Fig. 6. Therefore, the misalignment of the first grating 3 in the rotation directions around the direction X and the direction Y is adjusted as described above such that the unintended Moire fringes having a distortion can be eliminated, and thus detection of the unintended Moire fringes having a distortion by the detector 5 can be effectively significantly reduced or prevented.

According to the first embodiment, as described above, the controller 7 acquires the pitch D1 of the Moire fringes in the grating direction based on the Moire fringes, and acquires the misalignment amount $\Delta R$ of the first grating 3 in the X-ray irradiation axis direction based on the acquired pitch D1 of the Moire fringes in the grating direction. When the first grating 3 is misaligned in the X-ray irradiation axis direction, as described above, the Moire fringes having a pitch D1 in the grating direction occur. Therefore, as in the first embodiment, the pitch of the Moire fringes in the grating direction is acquired such that the misalignment amount ΔR of the first grating 3 in the X-ray irradiation axis direction can be accurately acquired using the relationship between the pitch D1 of the Moire fringes in the grating direction and the misalignment amount ΔR of the first grating 3 in the X-ray irradiation axis direction. Consequently, the misalignment of the first grating 3 in the X-ray irradiation axis direction can be accurately adjusted such that the unintended Moire fringes having a pitch D1 in the grating direction are eliminated.

According to the first embodiment, as described above, the controller 7 acquires the pitch D2 of the Moire fringes in the direction different from the grating direction based on the Moire fringes, and acquires the misalignment amount θ of the first grating 3 in the rotation direction around the X-ray irradiation axis direction based on the acquired pitch D2 of the Moire fringes in the direction different from the grating direction. When the first grating 3 is misaligned in the rotation direction around the X-ray irradiation axis direction, as described above, the unintended Moire fringes having a pitch D2 in the direction different from the grating direction occur. Therefore, as in the first embodiment, the pitch D2 of the Moire fringes in the direction different from the grating direction is acquired such that the misalignment amount θ of the first grating 3 in the rotation direction around the X-ray irradiation axis direction can be accurately acquired using the relationship between the pitch D2 of the Moire fringes in the direction different from the grating direction and the misalignment amount θ of the first grating 3 in the rotation direction around the X-ray irradiation axis direction. Consequently, the misalignment of the first grating 3 in a direction different from the X-ray irradiation axis direction can be accurately adjusted such that the unintended Moire fringes having a pitch in the direction different from the grating direction are eliminated.

According to the first embodiment, as described above, the controller 7 controls the adjustment mechanism 6 to adjust the misalignment of the first grating 3 in the rotation direction around the direction X and the rotation direction around the direction Y so as to eliminate the distortion of the Moire fringes. When the first grating 3 or the second grating 4 is misaligned in the rotation direction around the direction X or the rotation direction around the direction Y, as described above, the unintended Moire fringes having a distortion occur. Therefore, as in the first embodiment, the misalignment of the first grating 3 in the rotation direction around the direction X and the rotation direction around the direction Y is adjusted so as to eliminate the distortion of the Moire fringes such that unintended Moire fringes that occur due to the misalignment of the first grating 3 in the rotation direction around the direction X and the rotation direction around the direction Y can be easily eliminated.

(First Modified Example of First Embodiment)

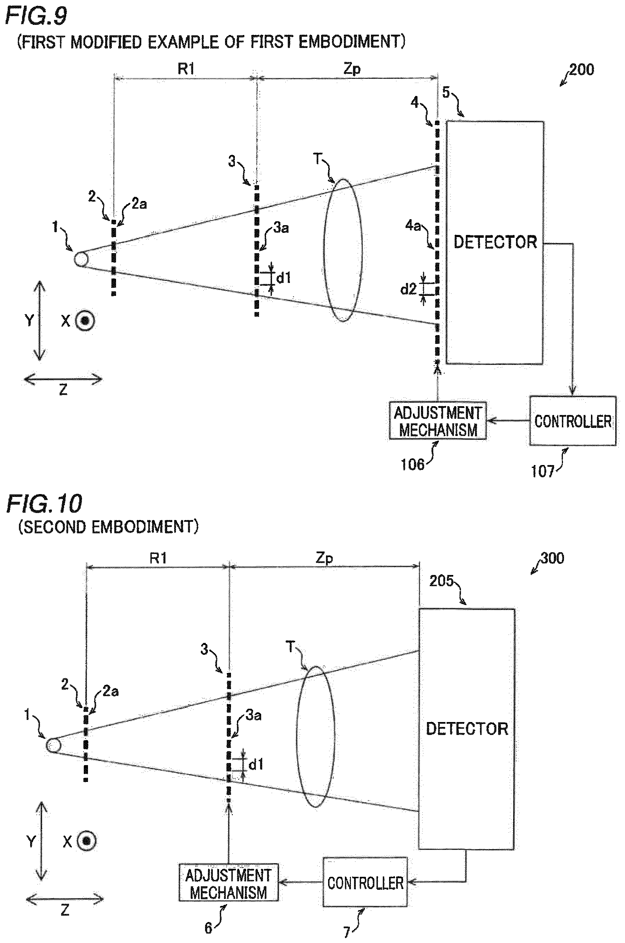

Next, a first modified example of the first embodiment is described with reference to Fig. 9.

As shown in Fig. 9, in an X-ray phase contrast imaging apparatus 200 according to the first modified example of the first embodiment, an adjustment mechanism 106 adjusts the position of a second grating 4 based on a control signal from a controller 107. In other words, the adjustment mechanism 106 adjusts a relative position between a first grating 3 and the second grating 4 based on the control signal from the controller 107.

The controller 107 controls the adjustment mechanism 106 to adjust the misalignment of the second grating 4 based on unintended Moire fringes detected by a detector 5. In other words, the controller 107 controls the adjustment mechanism 106 to adjust the relative position between the first grating 3 and the second grating 4 based on the unintended Moire fringes detected by the detector 5.

In this case, as in the aforementioned first embodiment, the adjustment mechanism 106 can adjust the misalignment of the second grating 4 in a rotation direction around a direction Z, a rotation direction around a direction X, and a rotation direction around a direction Y.

Regarding the misalignment of the second grating 4 in the direction Z, the misalignment amount of the second grating 4 in the direction Z cannot be obtained from the formulas (5) and (6) according to the aforementioned first embodiment.

In view of this, according to the first modified example of the first embodiment, the controller 107 acquires a distance Zpp between the first grating 3 and the second grating 4 in a misaligned state using the following formula (8).

[Mathematical Formula 8]

$$Zpp = \frac{d3 - d1}{d1} R1 \quad \cdots (8)$$

In the formula (8), whereas the pitch d1 of the first grating 3 and the pitch d3 of a self-image of the first grating 3 are known values, an actual distance R1 is unknown. Therefore, according to the first modified example of the first embodiment, a design value is used as the value of the distance R1 when the distance Zpp is acquired using the formula (8).

The controller 107 takes a difference between Zp, which is the design value of a distance between the first grating 3 and the second grating 4, and the distance Zpp between the first grating 3 and the second grating 4 in a misaligned state using the following formula (9) to acquire the misalignment amount ΔZp of the first grating 3 in the direction Z.

[Mathematical Formula 9]

$$Zp - Zpp = \Delta Zp \quad \cdots (9)$$

The controller 107 controls the adjustment mechanism 106 to move the second grating 4 along the direction Z by the misalignment amount $\Delta Zp$ so as to adjust the misalignment of the second grating 4 in the direction Z.

The remaining structures and effects of the first modified example of the first embodiment are similar to those of the aforementioned first embodiment.

[Second Embodiment]

Next, a second embodiment is described with reference to Fig. 10. In this second embodiment, an example in which a second grating is not provided is described unlike the aforementioned first embodiment in which the second grating is provided. The same structures as those of the aforementioned first embodiment are denoted by the same reference numerals in the drawings, and description thereof is omitted.

(Structure of X-ray Phase Contrast Imaging Apparatus)

As shown in Fig. 10, an X-ray phase contrast imaging apparatus 300 according to the second embodiment of the present invention is different from the X-ray phase contrast imaging apparatus 100 according to the aforementioned first embodiment in that the second grating 4 according to the first embodiment is not provided and a detector 205 is provided.

According to the first embodiment, the pitch of the detection elements of the detector 5 is larger than the pitch d3 of the self-image of the first grating 3, and thus it is difficult for the detector 5 to directly detect the self-image of the first grating 3. Therefore, according to the first embodiment, the second grating 4 is provided.

On the other hand, according to the second embodiment, the pitch of detection elements (not shown) of the detector 205 is smaller than the pitch d3 of a self-image of a first grating 3. Specifically, a pitch of the detection elements of the detector 205 is an integer fraction of the pitch d3 of the self-image of the first grating 3. Therefore, according to the second embodiment, the detector 205 can directly detect the self-image of the first grating 3. Thus, according to the second embodiment, the second grating 4 according to the first embodiment is not provided.

According to the second embodiment, the detector 205 is designed to be disposed at a position away from the first grating 3 by a Talbot distance Zp.

In the structure according to the second embodiment, similarly to the first embodiment, when the first grating 3 is misaligned from a design value, unintended Moire fringes occur due to a superposition of the self-image of the first grating 3 and the detector 205, as shown in Figs. 4 to 6.

Therefore, according to the second embodiment, the controller 7 controls the adjustment mechanism 6 to adjust the misalignment of the first grating 3 based on the unintended Moire fringes detected by the detector 205. In other words, the controller 7 controls the adjustment mechanism 6 to adjust a relative position between the first grating 3 and the detector 205 based on the unintended Moire fringes detected by the detector 205. A method for adjusting the misalignment of the first grating 3 is the same as that of the first embodiment, and thus description thereof is omitted.

The remaining structures of the second embodiment are similar to those of the aforementioned first embodiment.

(Effects of Second Embodiment)

According to the second embodiment, the following effects are achieved.

According to the second embodiment, as described above, the adjustment mechanism 6 that adjusts the position of the first grating 3 is provided. Furthermore, the controller 7 that controls the adjustment mechanism 6 to adjust the misalignment of the first grating 3 based on the Moire fringes detected by the detector 205 is provided. Thus, even when unintended Moire fringes occur, the misalignment of the first grating 3 can be adjusted based on the occurring unintended Moire fringes. Consequently, unintended Moire fringes can be eliminated, and thus detection of unintended Moire fringes by the detector 205 can be significantly reduced or prevented. Thus, similarly to the case of the X-ray phase contrast imaging apparatus 200 according to the aforementioned first embodiment, deterioration of the image quality of a captured image due to unintended Moire fringes can be significantly reduced or prevented.

The remaining effects of the second embodiment are similar to those of the aforementioned first embodiment.

[Modified Examples]

The embodiments disclosed this time must be considered as illustrative in all points and not restrictive. The scope of the present invention is not shown by the above description of the embodiments but by the scope of claims for patent, and all modifications (modified example) within the meaning and scope equivalent to the scope of claims for patent are further included.

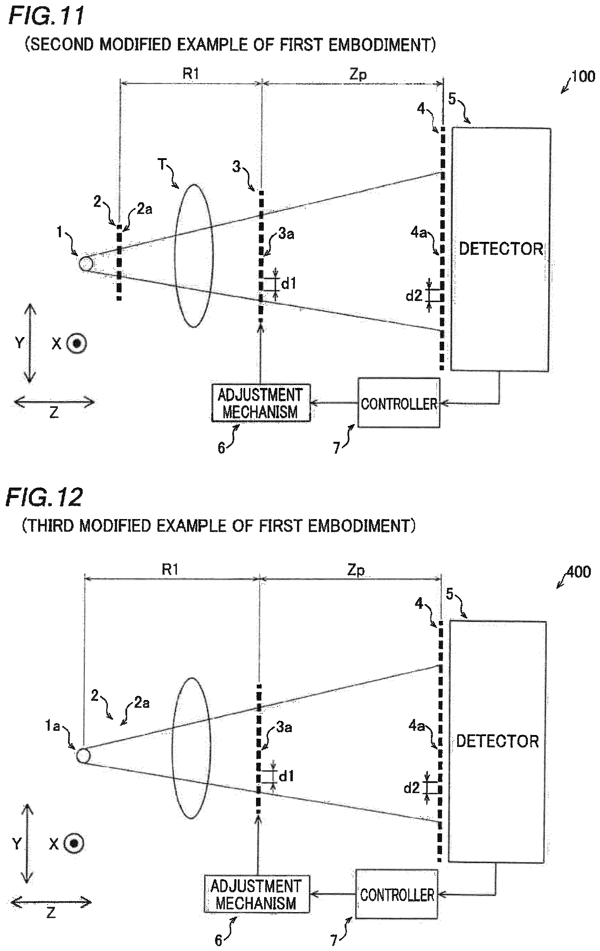

For example, while the example in which the subject is placed on the side of the first grating closer to the detector has been shown in each of the aforementioned first and second embodiments, the present invention is not restricted to this. According to the present invention, the subject may not be placed on the side of the first grating closer to the detector. For example, as in an X-ray phase contrast imaging apparatus 100 according to a second modified example of the first embodiment shown in Fig. 11, a subject T may be placed on the side of a first grating 3 opposite to a detector 5.

While the example in which the third grating that increases the coherence of the X-rays is provided in the X-ray phase contrast imaging apparatus has been shown in each of the aforementioned first and second embodiments, the present invention is not restricted to this. According to the present invention, the third grating that increases the coherence of the X-rays may not be provided in the X-ray phase contrast imaging apparatus. For example, as in an X-ray phase contrast imaging apparatus 400 according to a third modified example of the first embodiment shown in Fig. 12, a third grating may not be provided when an X-ray source 1a can radiate sufficiently coherent X-rays.

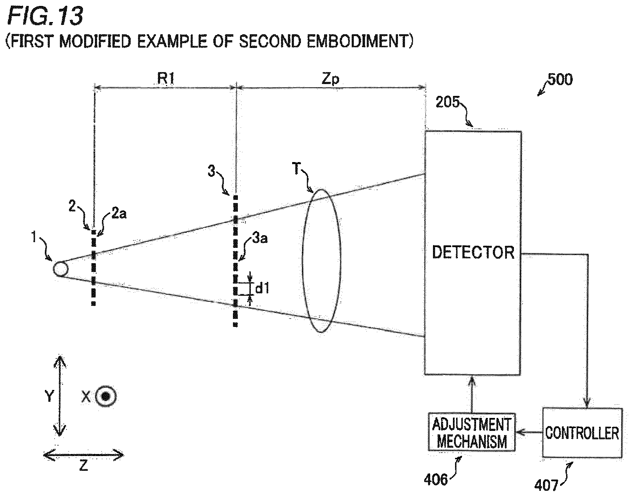

While the example in which the adjustment mechanism adjusts the position of the first grating, and the controller adjusts the misalignment of the first grating with the adjustment mechanism has been shown in the aforementioned second embodiment, the present invention is not restricted to this. According to the present invention, as in an X-ray phase contrast imaging apparatus 500 according to a first modified example of the second embodiment shown in Fig. 13, an adjustment mechanism 406 may adjust the position of a detector 205, and a controller 407 may adjust the misalignment of the detector 205 with the adjustment mechanism 406. Alternatively, according to the present invention, the adjustment mechanism may adjust both the position of the first grating and the position of the detector, and the controller may adjust both the misalignment of the first grating and the misalignment of the detector with the adjustment mechanism.

While the example in which the adjustment mechanism adjusts the position of the first grating, and the controller adjusts the misalignment of the first grating with the adjustment mechanism has been shown in the aforementioned first embodiment, the present invention is not restricted to this. According to the present invention, the adjustment mechanism may adjust both the position of the first grating and the position of the second grating, and the controller may adjust both the misalignment of the first grating and the misalignment of the second grating with the adjustment mechanism.

While the example in which the controller controls the adjustment mechanism to adjust the misalignment of the first grating such that the detector no longer detects unintended Moire fringes has been shown in each of the aforementioned first and second embodiments, the present invention is not restricted to this. According to the present invention, the controller may control the adjustment mechanism to adjust the misalignment of the first grating, the misalignment of the second grating, or the misalignment of the detector such that the pitch of the Moire fringes becomes a predetermined pitch. In this case, the pitch of the Moire fringes can be adjusted to a predetermined pitch suitable for imaging of the subject while detection of unintended Moire fringes by the detector is significantly reduced or prevented. This adjustment is effective when a captured image is acquired from a single Moire image (Moire fringes having a predetermined pitch suitable for imaging of the subject) by a Fourier transform method.

While the example in which the misalignment of the first grating in the direction Z (X-ray irradiation axis direction), the rotation direction around the direction Z (the rotation direction around the X-ray irradiation axis direction), and the rotation directions around the direction X (first orthogonal direction) and the direction Y (second orthogonal direction) is adjusted has been shown in each of the aforementioned first and second embodiments, the present invention is not restricted to this. According to the present invention, the misalignment of the first grating, the misalignment of the second grating, or the misalignment of the detector in at least one of the X-ray irradiation axis direction, the rotation direction around the X-ray irradiation axis direction, and the rotation directions around the first orthogonal direction and the second orthogonal direction orthogonal to each other in the plane orthogonal to the X-ray irradiation axis direction may be adjusted.

While the processing performed by the controller has been illustrated using a flow in a flow-driven manner in which processing is performed in turn along a processing flow for the convenience of illustration in the aforementioned first embodiment, the present invention is not restricted to this. According to the present invention, the processing performed by the controller may be performed in an event-driven manner in which processing is performed on an event basis. In this case, the processing may be performed in a complete event-driven manner or in a combination of an event-driven manner and a flow-driven manner.

Description of Reference Numerals

1, 1a X-ray source
3 first grating
4 second grating
5, 205 detector
6, 106, 406 adjustment mechanism
7, 107, 407 controller
100, 200, 300, 400, 500 X-ray phase contrast imaging apparatus

**Claims**

1. An X-ray phase contrast imaging apparatus comprising:

an X-ray source (1);
a first grating (3) irradiated with X-rays from the X-ray source to form a self-image;
a second grating (4) irradiated with the X-rays that have passed through the first grating;
a detector (5) that detects the X-rays that have passed through the second grating;
an adjustment mechanism (6) that adjusts a position of the first grating or a position of the second grating; and
a controller (7) that acquires a pitch of Moire fringes from an image of the Moire fringes detected by the detector and controls the adjustment mechanism to adjust a misalignment of the first grating or a misalignment of the second grating in an irradiation axis direction of the X-rays or a rotation direction around the irradiation axis direction of the X-rays based on the acquired pitch of the Moire fringes, and/or controls the adjustment mechanism to adjust the misalignment of the first grating,
wherein the controller acquires a misalignment amount of the first grating or a misalignment amount of the second grating in the irradiation axis direction of the X-rays or the rotation direction around the irradiation axis direction of the X-rays based on at least the acquired pitch of the Moire fringes and a pitch of the second grating, and
wherein the controller adjusts the misalignment of the first grating or the misalignment of the second grating in the irradiation axis direction of the X-rays or the rotation direction around the irradiation axis direction of the X-rays based on the misalignment amount of the first grating or the misalignment amount of the second grating in the irradiation axis direction of the X-rays or the rotation direction around the irradiation axis direction of the X-rays.

2. The X-ray phase contrast imaging apparatus according to claim 1, wherein
the controller controls the adjustment mechanism to adjust the misalignment of the first grating or the misalignment of the second grating in a rotation direction around a first orthogonal direction or a second orthogonal direction orthogonal to each other in a plane orthogonal to the irradiation axis direction of the X-rays based on variations of pixel values of the Moire fringes in a grating direction or a direction orthogonal to the grating direction detected by the detector.

3. The X-ray phase contrast imaging apparatus according to claim 1, wherein
the controller controls the adjustment mechanism to adjust the misalignment of the first grating or the misalignment of the second grating such that the detector no longer detects the Moire fringes or a pitch of the Moire fringes becomes a predetermined pitch.

4. The X-ray phase contrast imaging apparatus according to claim 1, wherein
the controller acquires the position of the first grating in the irradiation axis direction of the X-rays based on the pitch of the Moire fringes and the pitch of the second grating in the grating direction, and acquires the misalignment amount of the first grating or the misalignment amount of the second grating in the irradiation axis direction of the X-rays by acquiring a difference between the acquired position of the first grating and the position of the first grating in a non-misaligned state.

5. The X-ray phase contrast imaging apparatus according to claim 1, wherein
the controller acquires the pitch of the Moire fringes in a direction different from the grating direction based on the Moire fringes, and acquires a misalignment amount of the first grating or a misalignment amount of the second grating in the rotation direction around the irradiation axis direction of the X-rays based on the acquired pitch of the Moire fringes in the direction different from the grating direction.

6. The X-ray phase contrast imaging apparatus according to claim 5, wherein
the controller acquires an angle formed by the self-image of the first grating and the second grating in the rotation direction around the irradiation axis direction of the X-rays as a misalignment amount of the first grating or a misalignment amount of the second grating in the rotation direction around the irradiation axis direction of the X-rays based on the pitch of the Moire fringes in the direction different from the grating direction and a pitch of the second grating.

7. The X-ray phase contrast imaging apparatus according to claim 1, wherein
the controller controls the adjustment mechanism to adjust the misalignment of the first grating or the misalignment of the second grating in the rotation direction around the first orthogonal direction and the rotation direction around the second orthogonal direction so as to eliminate a distortion of the Moire fringes.

**Patentansprüche**

1. Röntgen-Phasenkontrast-Bildgebungseinrichtung, umfassend:

eine Röntgenstrahlenquelle (1);
ein erstes Gitter (3), das mit Röntgenstrahlen von der Röntgenstrahlenquelle bestrahlt wird, um ein Selbstbild zu bilden;
ein zweites Gitter (4), das mit den Röntgenstrahlen bestrahlt wird, die durch das erste Gitter hindurchgelangt sind;

einen Detektor (5), der Röntgenstrahlen erkennt, die durch das zweite Gitter hindurchgelangt sind;

einen Anpassungsmechanismus (6), der eine Position des ersten Gitters oder eine Position des zweiten Gitters anpasst; und

eine Steuer- bzw. Regelung (7), die einen Abstand von Moire-Streifen von einem Bild der Moire-Streifen erfasst, die von dem Detektor erkannt wurden, und den Anpassungsmechanismus steuert bzw. regelt, um eine Fehlausrichtung des ersten Gitters oder eine Fehlausrichtung des zweiten Gitters in einer Bestrahlungsachsenrichtung der Röntgenstrahlen oder einer Drehrichtung um die Bestrahlungsachsenrichtung der Röntgenstrahlen basierend auf dem erfassten Abstand der Moire-Streifen anzupassen, und/oder den Anpassungsmechanismus steuert bzw. regelt, um die Fehlausrichtung des ersten Gitters anzupassen,

wobei die Steuer- bzw. Regelung einen Fehlausrichtungsbetrag des ersten Gitters oder einen Fehlausrichtungsbetrag des zweiten Gitters in der Bestrahlungsachsenrichtung der Röntgenstrahlen oder der Drehrichtung um die Bestrahlungsachsenrichtung der Röntgenstrahlen basierend auf zumindest dem erfassten Abstand der Moire-Streifen und einem Abstand des zweiten Gitters erfasst, und

wobei die Steuer- bzw. Regelung die Fehlausrichtung des ersten Gitters oder die Fehlausrichtung des zweiten Gitters in der Bestrahlungsachsenrichtung der Röntgenstrahlen oder der Drehrichtung um die Bestrahlungsachsenrichtung der Röntgenstrahlen basierend auf dem Fehlausrichtungsbetrag des ersten Gitters oder dem Fehlausrichtungsbetrag des zweiten Gitters in der Bestrahlungsachsenrichtung der Röntgenstrahlen oder der Drehrichtung um die Bestrahlungsachsenrichtung der Röntgenstrahlen anpasst.

2. Röntgen-Phasenkontrast-Bildgebungseinrichtung nach Anspruch 1, wobei
die Steuer- bzw. Regelung den Anpassungsmechanismus steuert bzw. regelt, um die Fehlausrichtung des ersten Gitters oder die Fehlausrichtung des zweiten Gitters in einer Drehrichtung um eine erste orthogonale Richtung oder eine zweite orthogonale Richtung, die in einer Ebene orthogonal zueinander liegen, die orthogonal zu der Bestrahlungsachsenrichtung der Röntgenstrahlen ist, basierend auf Variationen von Pixelwerten der Moire-Streifen in einer Gitterrichtung oder einer Richtung, die orthogonal zu der Gitterrichtung liegt, die von dem Detektor erkannt wurde.

3. Röntgen-Phasenkontrast-Bildgebungseinrichtung nach Anspruch 1, wobei
die Steuer- bzw. Regelung den Anpassungsmechanismus steuert bzw. regelt, um die Fehlausrichtung des ersten Gitters oder die Fehlausrichtung des zweiten Gitters derart anzupassen, dass der Detektor die Moire-Streifen nicht mehr erkennt oder ein Abstand der Moire-Streifen ein vorbestimmter Abstand wird.

4. Röntgen-Phasenkontrast-Bildgebungseinrichtung nach Anspruch 1, wobei
die Steuer- bzw. Regelung die Position des ersten Gitters in der Bestrahlungsachsenrichtung der Röntgenstrahlen basierend auf dem Abstand der Moire-Streifen und dem Abstand des zweiten Gitters in der Gitterrichtung erfasst und den Fehlausrichtungsbetrag des ersten Gitters oder den Fehlausrichtungsbetrag des zweiten Gitters in der Bestrahlungsachsenrichtung der Röntgenstrahlen durch Erfassen eines Unterschieds zwischen der erfassten Position des ersten Gitters oder der Position des ersten Gitters in einem nicht fehlerhaft ausgerichteten Zustand erfasst.

5. Röntgen-Phasenkontrast-Bildgebungseinrichtung nach Anspruch 1, wobei
die Steuer- bzw. Regelung den Abstand der Moire-Streifen in einer Richtung, die sich von der Gitterrichtung unterscheidet, basierend auf den Moire-Streifen erfasst, und einen Fehlausrichtungsbetrag des ersten Gitters oder einen Fehlausrichtungsbetrag des zweiten Gitters in der Drehrichtung um die Bestrahlungsachsenrichtung der Röntgenstrahlen basierend auf dem erfassten Abstand der Moire-Streifen in der Richtung, die sich von der Gitterrichtung unterscheidet, erfasst.

6. Röntgen-Phasenkontrast-Bildgebungseinrichtung nach Anspruch 5, wobei
die Steuer- bzw. Regelung einen Winkel, der durch das Selbstbild des ersten Gitters und des zweiten Gitters in der Drehrichtung um die Bestrahlungsachsenrichtung der Röntgenstrahlen gebildet wird, als einen Fehlausrichtungsbetrag des ersten Gitters oder einen Fehlausrichtungsbetrag des zweiten Gitters in der Drehrichtung um die Bestrahlungsachsenrichtung der Röntgenstrahlen basierend auf dem Abstand der Moire-Streifen in der Richtung, die sich von der Gitterrichtung unterscheidet, und einem Abstand des zweiten Gitters erfasst.

7. Röntgen-Phasenkontrast-Bildgebungseinrichtung nach Anspruch 1, wobei
die Steuer- bzw. Regelung den Anpassungsmechanismus steuert bzw. regelt, um die Fehlausrichtung des ersten Gitters oder die Fehlausrichtung des zweiten Gitters in der Drehrichtung um die erste orthogonale Richtung und die Drehrichtung um die zweite orthogonale Richtung anzupassen, um so eine Verzerrung der Moire-Streifen zu beseitigen.

**Revendications**

1. Appareil d'imagerie à différence de phase de rayons X comprenant :

>   une source de rayons X (1) ;
>   un premier réseau (3) irradié de rayons X de la source de rayons X pour former une image automatique ;
>   un second réseau (4) irradié de rayons X qui ont traversé le premier réseau ;
>   un détecteur (5) qui détecte les rayons X qui ont traversé le second réseau ;
>   un mécanisme de réglage (6) qui règle une position du premier réseau ou une position du second réseau ; et
>   une unité de commande (7) qui acquiert un pas de franges de moiré d'une image des franges de moiré détectées par le détecteur et qui contrôle le mécanisme de réglage pour régler un désalignement du premier réseau ou un désalignement du second réseau dans une direction d'axe d'irradiation des rayons X ou une direction de rotation autour de la direction d'axe d'irradiation des rayons X basé sur le pas acquis des franges de moiré, et/ou qui contrôle le mécanisme de réglage pour régler le désalignement du premier réseau,
>   dans lequel l'unité de commande acquiert une quantité de désalignement du premier réseau ou une quantité de désalignement du second réseau dans la direction d'axe d'irradiation des rayons X ou la direction de rotation autour de la direction d'axe d'irradiation des rayons X basée sur au moins le pas acquis des franges de moiré et un pas du second réseau, et
>   dans lequel l'unité de commande règle le désalignement du premier réseau ou le désalignement du second réseau dans la direction d'axe d'irradiation des rayons X ou la direction de rotation autour de la direction d'axe d'irradiation des rayons X basée sur la quantité de désalignement du premier réseau ou la quantité de désalignement du second réseau dans la direction d'axe d'irradiation des rayons X ou la direction de rotation autour de la direction d'axe d'irradiation des rayons X.

2. Appareil d'imagerie à différence de phase de rayons X selon la revendication 1, dans lequel l'unité de commande contrôle le mécanisme de réglage pour régler le désalignement du premier réseau ou le désalignement du second réseau dans une direction de rotation autour d'une première direction orthogonale ou d'une seconde direction orthogonale orthogonale l'une à l'autre dans un plan orthogonal à la direction d'axe d'irradiation des rayons X basée sur des variations des valeurs de pixels des franges de moiré dans une direction de réseau ou une direction orthogonale à la direction de réseau détectée par le détecteur.

3. Appareil d'imagerie à différence de phase de rayons X selon la revendication 1, dans lequel l'unité de commande contrôle le mécanisme de réglage pour régler le désalignement du premier réseau ou le désalignement du second réseau de sorte que le détecteur ne détecte plus les franges de moiré ou un pas des franges de moiré devient un pas prédéterminé.

4. Appareil d'imagerie à différence de phase de rayons X selon la revendication 1, dans lequel l'unité de commande acquiert la position du premier réseau dans la direction d'axe d'irradiation des rayons X basée sur le pas des franges de moiré et le pas du second réseau dans la direction du réseau, et acquiert la quantité de désalignement du premier réseau ou la quantité de désalignement du second réseau dans la direction d'axe d'irradiation des rayons X en acquérant une différence entre la position acquise du premier réseau et la position du premier réseau dans un état non désaligné.

5. Appareil d'imagerie à différence de phase de rayons X selon la revendication 1, dans lequel l'unité de commande acquiert le pas des franges de moiré dans une direction différente de la direction du réseau basé sur les franges de moiré, et acquiert une quantité de désalignement du premier réseau ou une quantité de désalignement du second réseau dans la direction de rotation autour de la direction d'axe d'irradiation des rayons X basée sur le pas acquis des franges de moiré dans la direction différente de la direction du réseau.

6. Appareil d'imagerie à différence de phase de rayons X selon la revendication 5, dans lequel l'unité de commande acquiert un angle formé par l'image automatique du premier réseau et du second réseau dans la direction de rotation autour de la direction d'axe d'irradiation des rayons X comme une quantité de désalignement du premier réseau ou une quantité de désalignement du second réseau dans la direction de rotation autour de la direction d'axe d'irradiation des rayons X basée sur le pas des franges de moiré dans la direction différente de la direction de réseau et un pas du second réseau.

7. Appareil d'imagerie à différence de phase de rayons X selon la revendication 1, dans lequel

l'unité de commande contrôle le mécanisme de réglage pour régler le désalignement du premier réseau ou le désalignement du second réseau dans la direction de rotation autour de la première direction orthogonale et la direction de rotation autour de la seconde direction orthogonale de manière à éliminer une distorsion des franges de moiré.

## FIG.1
(FIRST EMBODIMENT)

## FIG.2

*FIG.3*

Z (OPTICAL AXIS DIRECTION)

*FIG.4*

AT TIME OF MISALIGNMENT IN DIRECTION Z (OPTICAL AXIS DIRECTION)

SECOND GRATING + SELF-IMAGE OF FIRST GRATING (PITCH IS DIFFERENT FROM DESIGN) → UNINTENDED MOIRE FRINGES

*FIG.5*

AT TIME OF MISALIGNMENT IN ROTATION DIRECTION AROUND DIRECTION Z

SECOND GRATING + SELF-IMAGE OF FIRST GRATING → UNINTENDED MOIRE FRINGES

EP 3 488 783 B1

# FIG.6

## AT TIME OF MISALIGNMENT IN ROTATION DIRECTION AROUND DIRECTION Y

4

SECOND GRATING

SELF-IMAGE OF
FIRST GRATING

UNINTENDED
MOIRE FRINGES

# FIG.7

POSITION
ADJUSTMENT

PIXEL
VALUE

POSITION

PIXEL
VALUE

POSITION

# FIG.8

GRATING POSITION ADJUSTMENT PROCESSING

```
                    START

              ┌──────────────┐
              │ UNINTENDED   │  S1
              │ MOIRE FRINGES│─── No ──┐
              │   READ?      │         │
              └──────────────┘         │
                  │ Yes                │
         ┌─────────────────────────┐ S2│
         │ READ DISTORTION OF      │   │
         │ UNINTENDED MOIRE FRINGES│   │
         └─────────────────────────┘   │
                  │                     │
              ┌──────────────┐  S3      │
              │ DISTORTION   │          │
              │ OF MOIRE     │─── No ──┐│
              │ FRINGES READ?│         ││
              └──────────────┘         ││
                  │ Yes                ││
   ┌─────────────────────────────────┐ S4
   │ ADJUST GRATING POSITION          ││
   │ · ROTATION DIRECTION AROUND      ││
   │   DIRECTION Y                    ││
   │ · ROTATION DIRECTION AROUND      ││
   │   DIRECTION X                    ││
   └─────────────────────────────────┘│
                  │◄──────────────────┘│
         ┌─────────────────────────┐ S5│
         │ READ PITCH OF MOIRE      │   │
         │ FRINGES                  │   │
         └─────────────────────────┘   │
                  │                     │
              ┌──────────────┐  S6      │
              │ PITCH        │          │
              │ OF MOIRE     │─── No ──┐│
              │ FRINGES READ?│         ││
              └──────────────┘         ││
                  │ Yes                ││
   ┌─────────────────────────────────┐ S7
   │ ACQUIRE MISALIGNMENT AMOUNT IN   ││
   │ DIRECTION Z AND MISALIGNMENT     ││
   │ AMOUNT IN ROTATION DIRECTION     ││
   │ AROUND DIRECTION Z               ││
   └─────────────────────────────────┘│
                  │                 S8 ││
   ┌─────────────────────────────────┐││
   │ ADJUST GRATING POSITION          │││
   │ · DIRECTION Z                    │││
   │ · ROTATION DIRECTION AROUND      │││
   │   DIRECTION Z                    │││
   └─────────────────────────────────┘││
                  │◄──────────────────┘│
                  │◄──────────────────┘

                    END
```

## FIG.9
### (FIRST MODIFIED EXAMPLE OF FIRST EMBODIMENT)

DETECTOR

ADJUSTMENT MECHANISM

CONTROLLER

## FIG.10
### (SECOND EMBODIMENT)

DETECTOR

ADJUSTMENT MECHANISM

CONTROLLER

## FIG.11
(SECOND MODIFIED EXAMPLE OF FIRST EMBODIMENT)

## FIG.12
(THIRD MODIFIED EXAMPLE OF FIRST EMBODIMENT)

## FIG.13
(FIRST MODIFIED EXAMPLE OF SECOND EMBODIMENT)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014030115 A
- US 2011243300 A1
- US 2012163537 A1